# EUROPEAN PATENT SPECIFICATION

(11) **EP 1 299 380 B1**
(45) Date of publication and mention of the grant of the patent: **09.06.2004**
(21) Application number: 01940241.1
(22) Date of filing: 13.06.2001
(51) Int. Cl.: C07D 401/14, C07D 413/14, A61K 31/4045, A61K 31/4523, A61K 31/47, A61K 31/538, A61P 25/06, A61P 25/18, A61P 25/20, A61P 25/22, A61P 25/24, A61P 25/28

(54) **INDOLE DERIVATIVES USEFUL FOR THE TREATMENT OF CNS DISORDERS**
INDOLDERIVATE ZUM GEBRAUCH IN DER BEHANDLUNG VON ZNS-STÖRUNGEN
DERIVES INDOLIQUES UTILES POUR LE TRAITEMENT DES TROUBLES DU SYSTEME NERVEUX CENTRAL

(30) Priority: 29.06.2000 DK 200001018
(43) Date of publication of application: 09.04.2003
(73) Proprietor: H. LUNDBECK A/S, 2500 Copenhagen (DK)
(72) Inventor: BANG-ANDERSEN, Benny, DK-22 Kobenhavn N (DK); FELDING, Jakob, DK-2300 Kobenhavn S (DK); KEHLER, Jan, DK-2800 Kgs. Lyngby (DK)
(74) Representative: Nielsen, Leila
(86) International application number: PCT/DK2001/000407
(87) International publication number: WO 2002/000645

(56) References cited:
- WO-A1-94/20497
- WO-A1-98/28293
- US-B1- 6 262 087
- B.-J. CAO ET AL.: 'Influence of 5-HT 1A receptor antagonism on plus-maze behaviour in mice. II WAY 100635, SDZ 216-525 and NAN-190' PHARMACOLOGY BIOCHEMISTRY AND BEHAVIOR vol. 58, no. 2, 1997, pages 593 - 603, XP002948841

## Description

### Field of the Invention

The present invention relates to a novel class of indole derivatives having affinity for the dopamine D₄ receptor. The compounds are therefore useful in the treatment of certain psychiatric and neurologic disorders, in particular psychoses. The compounds also have affinity for the 5-HT_{2A} receptor.

### Background of the Invention

Dopamine D₄ receptors belong to the dopamine D₂ subfamily of receptors, which is considered to be responsible for the antipsychotic effect ofneuroleptics. The side effects of neuroleptic drugs, which primarily exert their effect via antagonism of D₂ receptors, are known to be due to D₂ receptor antagonism in the striatal regions of the brain. However, dopamine D₄ receptors are primarily located in areas of the brain other than striatum, suggesting that antagonists of the dopamine D₄ receptor will be devoid of extrapyramidal side effects. This is illustrated by the antipsychotic clozapine, which exerts higher affinity for D₄ than D₂ receptors and is lacking extrapyramidal side effects (Van Tol et al. *Nature* **1991,** *350*, 610; Hadley *Medicinal Research Reviews* **1996,** *16*, 507-526 and Sanner *Exp. Opin. Ther. Patents* **1998,** *8*, 383-393).

A number of D₄ ligands, which were postulated to be selective D₄ receptor antagonists (L-745,879 and U-101958), have been shown to posses antipsychotic potential (Mansbach et al. *Psychopharmacology* **1998** *135*, 194-200). However, recently it has been reported that these compounds are partial D₄ receptor agonists in various *in vitro* efficacy assays (Gazi et al. *Br. J. Pharmacol*. **1998,** *124*, 889-896 and Gazi et al. *Br. J. Pharmacol*. **1999,** *128*, 613-620). Furthermore, it was shown that clozapine, which is an effective antipsychotic, is a silent antagonists (Gazi et al. *Br. J. Pharmacol*. **1999,** *128*, 613-620).

Consequently, D₄ ligands, which are partial D₄ receptor agonists or antagonists, may have beneficial effects against psychoses.

Dopamine D₄ antagonists may also be useful for the treatment of cognitive deficits (Jentsch et al. *Psychopharmacology* **1999,** *142*, 78-84).

Furthermore, evidence for a genetic association between the "primarily inattentive" subtype of attention deficit hyperactivity disorder and a tandem duplication polymorphism in the gene encoding the dopamine D₄ receptor has been published (McCracken et al. *Mol*. *Psychiat*. **2000,** 5, 531-536). This clearly indicates a link between the dopamine D₄ receptor and attention deficit hyperactivity disorder and ligands affecting this receptor may be useful for the treatment of this particular disorder

Various effects are known with respect to compounds, which are ligands at the different serotonin receptor subtypes. As regards the 5-HT_{2A} receptor, which was previously referred to as the 5-HT₂ receptor, the following effects have been reported, e.g.:

Antidepressive effect and improvement of the sleep quality (Meert et al. *Drug. Dev. Res.* **1989,** *18*, 119), reduction of the negative symptoms of schizophrenia and of extrapyramidal side effects caused by treatment with classical neuroleptics in schizophrenic patients (Gelders *British J*. *Psychiatry* **1989**, *155* (suppl. 5), 33). Furthermore, selective 5-HT_{2A} antagonists could be effective in the prophylaxis and treatment of migraine (Scrip Report; "Migraine ― Current trends in research and treatment"; PJB Publications Ltd.; May 1991) and in the treatment of anxiety (Colpart et al. *Psychopharmacology* **1985,** *86*, 303-305 and Pecregaard et al. *Current Opinion in Therapeutic Patents* **1993**, *1*, 101-128).

Some clinical studies implicate the 5-HT₂ receptor subtype in aggressive behaviour. Furthermore, atypical serotonin-dopamine antagonist necroleptics have 5-HT₂ receptor antagonistic effect in addition to their dopamine blocking properties and have been reported to possess anti-aggressive behaviour (Connor et al. *Exp. Opin. Ther. Patents.* **1998,** *8(4)*, 350-351).

Recently, evidence has also accumulated which support the rational for selective 5-HT_{2A} antagonists as drugs capable of treating positive symptoms of psychosis (Lxysen et al. *Current Pharmaceutical Design* **1997,** *3*, 367-390 and Carlsson *Current Opinion in CPNS Investigational Drugs* **2000,** *2*(1), 22-24).
Accordingly, dopamine D₄ receptor ligands are potential drugs for the treatment of schizophrenia and other psychoses and compounds with combined effects at dopamine D₄ and 5-HT_{2A} receptors may have the further benefit of improved effect on positive and negative symptoms in schizophrenia, including depressive and anxiety symptoms. Dopamine D₄ ligands related to the compounds of the invention are known from WO 98/28293. The indane and dihydroindole derivatives disclosed herein have the general formula 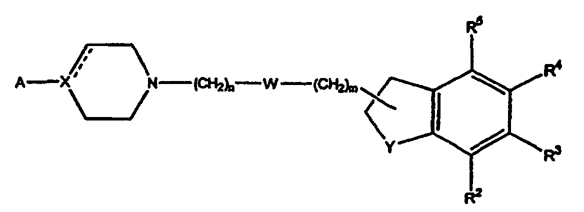 wherein A is an indole and Y is a group completing an indane or a dihydroindole and the other substituents are as defined in the application.

WO 94/18197, EP 329168, WO 93/16073, EP 732332, WO 98/37893 and WO 95/11680 disclose compounds, claimed to be dopamine D₄ ligands useful as antipsychotics, which, like the compounds of the present invention, have a tetrahydroquinonnone or a tetrahydroisoquinolinone in one end of the molecule. However, these compounds do not contain an indole as the compounds of the present invention The compounds of WO 93/16073 are also said to have antagonistic activity at 5-HT₂ receptors. WO 98/28293 discloses substituted indane or dihydroindole compounds, which have high affinity for D₄ receptors. Some of the compounds also have affinity for 5-HT receptors and/or the 5-HT transporter.

### Summary of the Invention

The object of the present invention is to provide compounds, which are partial agonists or antagonists at the dopamine D₄ receptor, in particular compounds with combined effects at the dopamine D₄ receptor and the 5-HT_{2A} receptor.

Accordingly, the present invention relates to novel compounds of formula I 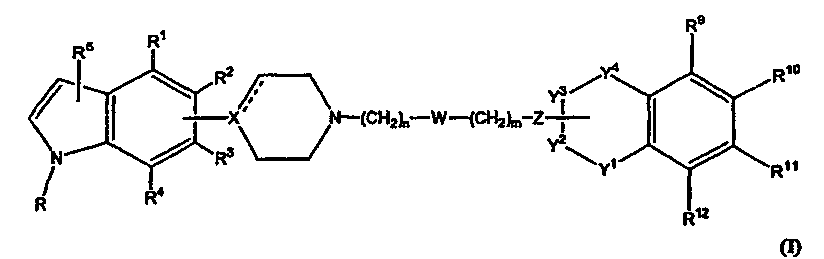 wherein
a) Y¹ is N, which is bound to Z, Z and Y² are selected from CH₂, CO, CS, SO and SO₂; provided that at least one of Z and Y² is CH₂; Y³ is O, S or CHR⁷ and Y⁴ is O, S or CHR⁸, provided that only one of Y³ and Y⁴ is O or S;
b) Y² is N, which is bound to Z, Z and Y¹ are selected from CH₂, CO, CS, SO and SO₂; provided that at least one of Z and Y¹ is CH₂; Y³ is CHR⁷ and Y⁴ is O, S or CHR⁸;
c) Y² is N, which is bound to Z, Z and Y³ are selected from CH₂, CO, CS, SO and SO₂ provided that at least one of Z and Y³ is CH₂; Y¹ is CHR⁶ and Y⁴ is O, S or CHR⁸;

W is a bond, O, S, CO, CS, SO or SO₂;
n is 0-5, m is 0-5 and n + m is 1-6; provided that when W is O or S, then n ≥ 2 and m ≥ 1; when W is CO, CS, SO or SO₂, then n ≥ 1and m ≥ 1;
X is C, CH or N, provided that when X is C, the dotted line indicates a bond, and when X is N or CH, the dotted line indicates no bond;
one of R¹, R², R³ and R⁴ forms a bond to X and the others of R¹, R², R³, R⁴ and R⁵ and R⁹-R¹² are independently selected from hydrogen, halogen, cyano, nitro, amino, hydroxy, C₁₋₆-alkyl-amino, di-(C₁₋₆-alkyl)-amino, C₁₋₆-alkyl, C₂₋₆-alkenyl, C₂₋₆-alkynyl, C₁₋₆-alkoxy, C₁₋₆-alkylthio, C₁₋₆-alkyl substituted with hydroxy or thiol, C₃₋₈-cycloalkyl, C₃₋₈-cycloalkyl-C₁₋₆-alkyl, acyl, thioacyl, trifluoromethyl, trifluoromethylsulfonyl or C₁₋₆ alkylsulfonyl;
R⁶, R⁷ and R⁸ are hydrogen or halogen;
R is hydrogen, C₁₋₆-alkyl, C₂₋₆-alkenyl, C₂₋₆-alkynyl, C₁₋₆-alkyl substituted with hydroxy or thiol, C₃₋₈-cycloalkyl, C₃₋₈-cycloalkyl-C₁₋₆-alkyl, acyl, thioacyl, trifluoromethylsulfonyl and C₁₋₆-alkylsulfonyl;
or pharmaceutically acceptable salts thereof.

In a first embodiment, the present invention relates to compounds wherein Y¹ is N, which is bound to Z, Z and Y² are selected from CH₂, CO, CS, SO and SO₂; provided that at least one of Z and Y² is CH₂; Y³ is O, S or CHR⁷, and Y⁴ is O, S or CHR⁸, provided only one of Y³ and Y⁴ is O or S.

Particular compounds of the invention are compounds wherein:
Y¹ is N, which is bound to Z, Z and Y² are selected from CH₂ and CO; provided that at least one of Z and Y² is CH₂; Y³ is CHR⁷ and Y⁴ is O, S or CHR⁸;
Y¹ is N, which is bound to Z, Z is CH₂, Y² is CO, Y³ is CHR⁷ and Y⁴ is O, S or CHR⁸;
Y¹ is N, which is bound to Z, Z and Y² are CH₂; Y³ is CHR⁷ and Y⁴ is O, S or CHR⁸; and
Y¹ is N, which is bound to Z, Z is CO, Y² is CH₂, Y³ is CHR⁷ and Y⁴ is O, S or CHR⁸.

In a second embodiment, the present invention relates to compounds wherein Y² is N, which is bound to Z, Z and Y¹ are selected from CH₂, CO, CS, SO and SO₂; provided that at least one of Z and Y¹ is CH₂; Y³ is CHR⁷ and Y⁴ is O, S or CHR⁸:

Particular compounds of the invention are compounds wherein:
Y² is N, which is bound to Z, Z and Y¹ are selected from CH₂ and CO; provided that at least one of Z and Y¹ is CH₂; Y³ is CHR⁷ and Y⁴ is O, S or CHR⁸;
Y² is N, which is bound to Z, Z is CH₂ and Y¹ is CO; Y³ is CHR⁷ and Y⁴ is O, S or CHR⁸; Y² is N, which is bound to Z, Z and Y¹ are CH₂; Y³ is CHR⁷ and Y⁴ is O, S or CHR⁸; and
Y² is N, which is bound to Z, Z is CO and Y¹ is CH₂; Y³ is CHR⁷ and Y⁴ is O, S or CHR⁸.

In a third embodiment, the present invention relates to compounds wherein Y² is N, which is bound to Z, Z and Y³ are selected from CH₂, CO, CS, SO and SO₂ provided that at least one of Z and Y³ is CH₂; Y¹ is CHR⁶ and Y⁴ is O, S or CHR⁸,

Particular compounds of the invention are compounds wherein:
Y² is N, which is bound to Z, Z and Y³ are selected from CH₂ and CO; Y¹ is CHR⁶ and Y⁴ is O, S or CHR⁸;
Y² is N, which is bound to Z, Z is CH₂ and Y³ is CO; Y¹ is CHR⁶ and Y⁴ is O, S or CHR⁸;
Y² is N, which is bound to Z, Z and Y³ is CH₂; Y¹ is CHR⁶ and Y⁴ is O, S or CHR⁸: and
Y² is N, which is bound to Z, Z is CO and Y³ is CH₂; Y¹ is CHR⁶ and Y⁴ is O, S or CHR⁸.

In a particular embodiment, the present invention relates to compounds wherein R² or R³ form a bond to X.

In other embodiments, the invention relates to compounds wherein X is N, C or CH, respectively.

In another specific embodiment, the invention relates to such compounds wherein one of R¹, R², R³ and R⁴ forms a bond to X and the others of R¹, R², R³,R⁴ and R⁵ and R⁹-R¹² are selected from hydrogen, halogen, cyano, nitro, amino, C₁₋₆-alkyl, C₁₋₆-alkoxy, C₁₋₆-alkylthio, hydroxy and trifluoromethyl and R is hydrogen, C₁₋₆-alkyl, or C₁₋₆-alkylcarbonyl.

In a particular embodiment, R⁶, R⁷ and R⁸ are hydrogen in the above compounds of the invention.

In another particular embodiment, R, R¹-R⁵, and R⁹-R¹² are selected from hydrogen and halogen in the above compounds of the invention. More particularly, R¹ to R⁴ are selected from hydrogen and flour.

In another embodiment, W is a bond and n+m is 2 to 4 in the above compounds of the invention.

Specific compounds of the invention may be selected from
5-{4-[2-(2-Oxo-3,4-dihydro-2*H*-quinolin-1-yl)ethyl]piperazin-1-yl}-1*H*-indole,
5-{4-[3-(2-Oxo-3,4-dihydro-2*H*-quinolin-1-yl)propan-1-yl]piperazin-1-yl}-1*H*-indole,
5-{4-[4-(2-Oxo-3,4-dihydro-2*H*-quinolin-1-yl)butan-1-yl]piperazin-1-yl}-1*H*-indole,
6-{4-[3-(2-Oxo-3,4-dihydro-2*H*-quinolin-1-yl)propan-1-yl]piperazin-1-yl}-1*H*-indole,
6-{4-[4-(2-Oxo-3,4-dihydro-2*H*-quinolin-1-yl)butan-1-yl]piperazin-1-yl}-1*H*-indole
5-{4-[3-(2-Oxo-3,4-dihydro-2*H*-quinolin-1-yl)propan-1-yl]-3,6-dihydro-2*H*-pyridin-4-yl}-1H-indole,
5-{4-[3-(2-Oxo-3,4-dihydro-2*H*-quinolin-1-yl)propan-1-yl]piperidin-4-yl}-1*H*-indole,
5-{4-[4-(3-Oxo-3,4-dihydro-2*H*-1,4-benzoxazin-4-yl)butan-1-yl]piperazin-1-yl}-1*H*-indole,
5-{4-[3-(1-Oxo-3,4-dihydro-1*H*-quinolin-2-yl)propan-1-yl]piperazin-1-yl}-1*H*-indole,
5-{4-[4-(1-Oxo-3,4-dihydro-1*H*-quinolin-2-yl)butan-1-yl]piperazin-1-yl}-1*H*-indole,
5-{4-[3-(3,4-Dihydro-2*H*-quinolin-1-yl)propan-1-yl]piperazin-1-yl}-1*H*-indole,
5-{4-[3-(3,4-Dihydro-2*H*-quinolin-1-yl)propan-1-yl]piperazin-1-yl}-1*H*-indole,
5-{4-[3-(3,4-dihydro-1*H*-isoquinolin-2-yl)-3-oxopropan-1-yl]piperazin-1-yl}-1*H*-indole, and
5-{4-[4-(3,4-dihydro-1*H*-isoquinolin-2-yl)-4-oxobutan-1-yl]piperazin-1-yl}-1*H*-indole, or pharmaceutically acceptable salts thereof.

The compounds of the invention have been found to show high affinity for the dopamine D₄ receptor and to be partial agonists or antagonists at this receptor. The compounds also show affinity for serotonergic 5-HT_{2A} receptors.

Accordingly, the compounds of the invention are considered useful in the treatment of positive and negative symptoms of schizophrenia, other psychoses, anxiety disorders, such as generalised anxiety disorder, panic disorder and obsessive compulsive disorder, depression, aggression, cognitive disorders, side effects induced by conventional antipsychotic agents, migraine, attention deficit hyperactivity disorder and in the improvement of sleep.

In another aspect, the present invention provides a pharmaceutical composition comprising at least one compound of formula I as defined above or a pharmaceutically acceptable acid addition salt thereof in a therapeutically effective amount preferably in combination with one or more pharmaceutically acceptable carriers or diluents.

In a further aspect, the present invention provides the use of a compound of formula I as defined above or a pharmaceutically acceptable acid addition salt thereof for the manufacture of a pharmaceutical preparation for the treatment of the above mentioned disorders.

### Detailed Description of the Invention

The compounds of general formula I may exist as optical isomers thereof and such optical isomers are also embraced by the invention.

The term C₁₋₆-alkyl refers to a branched or unbranched alkyl group having from one to six carbon atoms inclusive, such as methyl, ethyl, 1-propyl, 2-propyl, 1-butyl, 2-butyl, 2-methyl-2-propyl,2-methyl-1-propyl, pentyl and hexyl.

Similarly, C₂₋₆-alkenyl and C₂₋₆-alkynyl, respectively, designate such groups having from two to six carbon atoms, including one double bond and triple bond, respectively, such as ethenyl, propenyl, butenyl, ethynyl, propynyl and butynyl.

The terms C₁₋₆-alkoxy, C₁₋₆-alkylthio, C₁₋₆-alkylsulfonyl, C₁₋₆-alkylamino,
C₁₋₆-alkylcarbonyl, etc. designate such groups in which the alkyl group is C₁₋₆-alkyl as defined above.

The term C₃₋₈-cycloalkyl designates a monocyclic or bicyclic carbocycle having three to eight C-atoms, such as cyclopropyl, cyclopentyl, cyclohexyl, etc.
Halogen means fluoro, chloro, bromo or iodo.

As used herein, the term acyl refers to a formyl, C₁₋₆-alkylcarbonyl, arylcarbonyl, aryl-C₁₋₆-alkylcarbonyl, C₃₋₈-cycloalkylcarbonyl or a C₃₋₈-cycloalkyl-C₁₋₆-alkyl-carbonyl group and the term thioacyl is the corresponding acyl group in which the carbonyl group is replaced with a thiocarbonyl group.

The term aryl refers to a carbocyclic aromatic group, such as phenyl, naphthyl, in particular phenyl, including methyl substituted phenyl or naphthyl.

The acid addition salts of the compounds of the invention are pharmaceutically acceptable salts formed with non-toxic acids. Exemplary of such organic salts are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic and theophylline acetic acids, as well as the 8-halotheophyllines, for example 8-bromotheophylline. Exemplary of such inorganic salts are those with hydrochloric, hydrobromic, sulfuric, sulfamic, phosphoric and nitric acids.

The pharmaceutical compositions of this invention, or those which are manufactured in accordance with this invention, may be administered by any suitable route, for example orally in the form of tablets, capsules, powders, syrups, etc., or parenterally in the form of solutions for injection. For preparing such compositions, methods well known in the art may be used, and any pharmaceutically acceptable carriers, diluents, excipients or other additives normally used in the art may be used.

Conveniently, the compounds of the invention are administered in unit dosage form containing said compounds in an amount of about 0.01 to 100 mg.

The total daily dose is usually in the range of about 0.05 - 500 mg, and most preferably about 0.1 to 50 mg of the active compound of the invention.

The compounds of the invention may be prepared as follows:
1) Alkylating a piperazine, piperidine or tetrahydropyridine of formula II with an alkylating derivative of formula III: 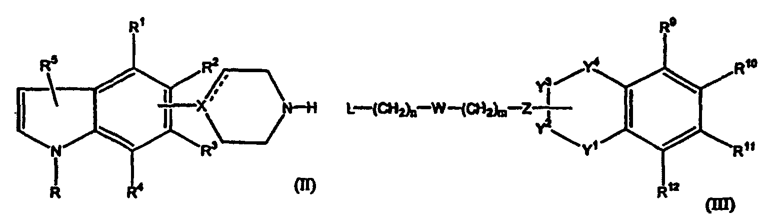 wherein R, R¹-R⁵, R⁹-R¹², Y¹-Y⁴, X, Z, n, m, W and the dotted line are as previously defined, and L is a leaving group such as e.g. halogen, mesylate or tosylate;
2) Reductive alkylation of an amine of formula II with a reagent of formula IV: 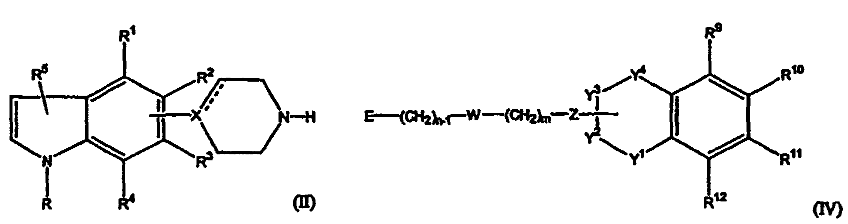 wherein R, R¹-R⁵, R⁹-R¹², Y¹-Y⁴, X, Z, n, m, W and the dotted line are as previously defined, and E is either an aldehyde or an activated carboxylic acid group;
3) Alkylating a compound of formula VI with an alkylating derivative of formula V: 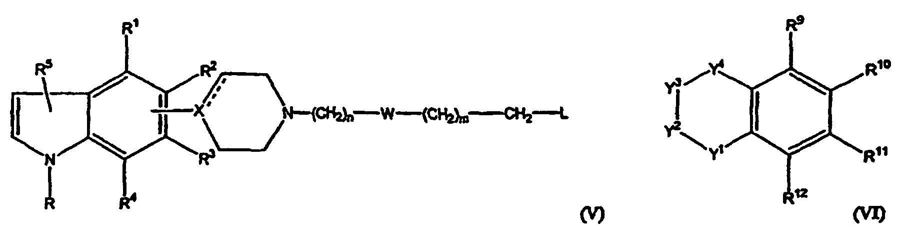 wherein X, R, R¹- R⁵, R⁹-R¹², n, m, W and the dotted line are as previously defined and L is a leaving group such as e.g. halogen, mesylate or tosylate, and Y¹-Y⁴ are as previously defined with the exception that the one of Y¹-Y², which is to be the point of attachment, is NH or N⁻;
4) Reducing the tetrahydropyridinyl double bond in derivatives of formula VII: 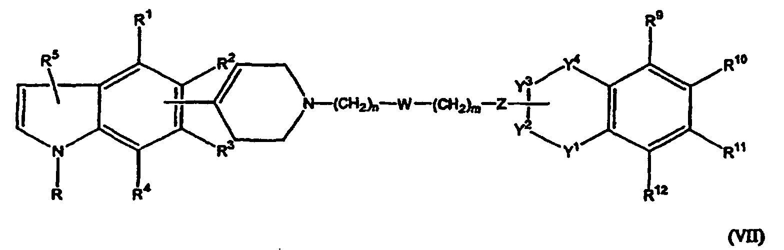 wherein R, R¹-R⁵, R⁹-R¹², Y¹-Y⁴, Z, n, m, and W are as previously defined;
5) Reducing the amide carbonyl in a compound of formula VIII: 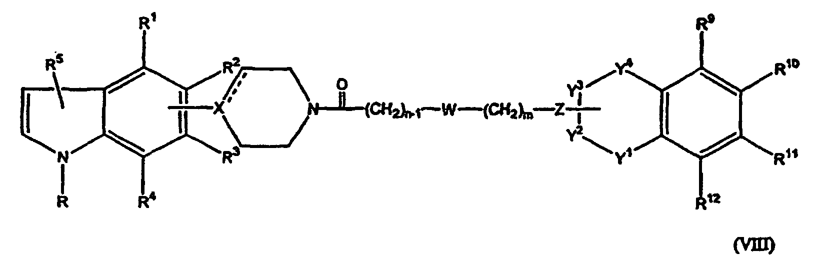 wherein R, R¹-R⁵, R⁹-R¹², Y¹-Y⁴, X, n, m, W, Z and the dotted line are as previously defined;
6) Reducing the amide carbonyl in a compound of formula IX: 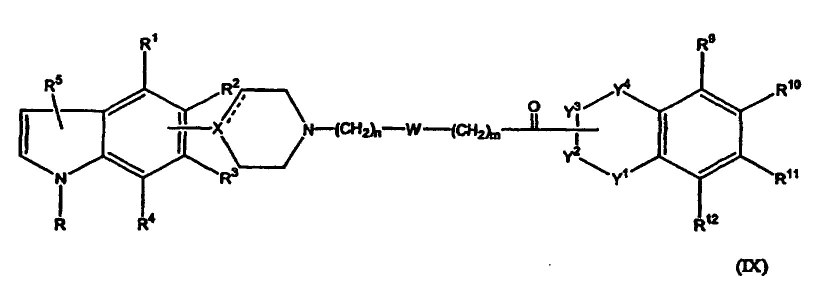 wherein R, R¹-R⁵, R⁹-R¹², Y¹-Y⁴, X, n, m, W and the dotted line are as previously defined;
7) Reductive alkylation of an amine of formula XI with a reagent of formula X: 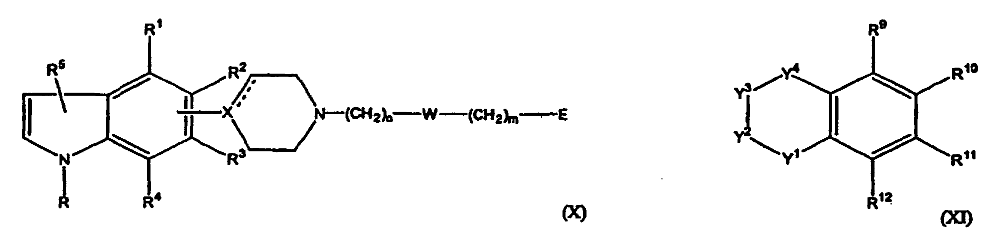 wherein X, R, R¹-R⁵, R⁹-R¹², m, n, W and the dotted line are as previously defined, and E is either an aldehyde or an activated carboxylic acid group and Y¹-Y⁴ are as previously defined with the exception that the one of Y¹-Y², which is to be the point of attachment, is NH and the ring member adjacent to NH is CH₂;
8) Acylation of an amine of the formula XI with a reagent of formula X 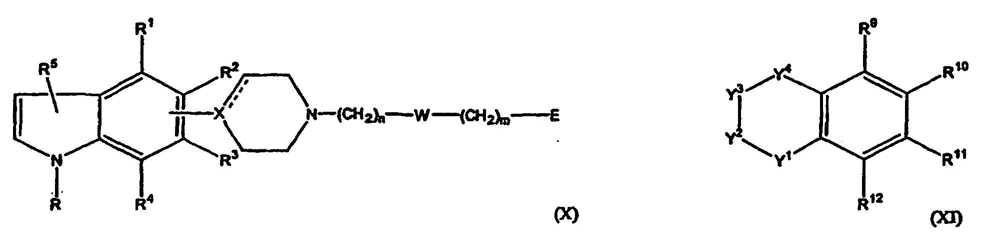
wherein X, R, R¹-R⁵, R⁹-R¹², m, n, W and the dotted line are as previously defined, and E is either an aldehyde or an activated carboxylic acid group and Y¹-Y⁴ are as previously defined with the exception that one of Y¹-Y², which is to be the point of attachment, is NH and the ring member adjacent to NH is CH₂; whereupon the compound of Formula (I) is isolated as the free base or a pharmaceutically acceptable acid addition salt thereof.

The alkylation according to methods 1) and 3) is conveniently performed in an inert organic solvent such as a suitably boiling alcohol or ketone, preferably in the presence of an organic or inorganic base (potassium carbonate, diisopropylethylamine or triethylamine) at reflux temperature. Alternatively, the alkylation can be performed at a fixed temperature, which is different from the boiling point, in one of the above mentioned solvents or in dimethyl formamide (DMF), dimethylsulfoxide (DMSO) or *N*-methylpyrrolidin-2-one (NMP), preferably in the presence of a base.

Some of the amines of formula (II) are known from the literature or may be prepared analogously (see WO 98/28293, US patent No. 5.576319 or WO 94/20459). Piperazines of formula (II) may be prepared from nitroindoles by reduction of the nitro group to an aniline, which subsequently is subjected to piperazine synthesis by methods obvious to a chemist skilled in the art (see also Kruse et al. *Recl.Trav.Chim., Pays-Bas*. **1988,** *107*, 303-309). Piperidines such as 5-(piperidin-4-yl)-1*H*-indoles may be prepared from the corresponding tetrahydropyridines (WO 94/20459). Alkylating reagents of formula (III) are known from the literature (see Oshiro et al. *J. Med. Chem.* **2000,** *43*, 177-189 and EP-B1-512525) or they can be prepared by methods obvious to a chemist skilled in the art by an analogous synthetic sequence (see Kowalski et al. *J.Heterocyclic Chem.* **2000,** *37*, 187-189, Mokrosz et al. *Pharmazie* **1997,** *52*, 423-428 and Misztal et al. *Med.Chem.Res.* **1992**, *2*, 82-87). Alkylating reagents of formula (V) can be prepared by methods obvious to a chemist skilled in the art, and compounds of formula (VI) are commercially available or described in the literature.

The reductive alkylation according to methods 2) and 7) is performed by standard literature methods. The reaction can be performed in two steps, e.g. coupling of derivatives of formula (II/XI) and the reagent of formula (IV/X) by standard methods via the carboxylic acid chloride or by use of coupling reagents such as e.g. dicyclohexyl carbodiimide followed by reduction of the resulting amide with lithium aluminium hydride or alane. The reaction can also be performed by a standard one-pot procedure. Aldehydes or carboxylic acids of formula (IV/X) can be prepared analogously to the synthetic sequence described for alkylating reagents of formula (III/V), but by the use of acetal protected haloalkanal derivatives or the corresponding protected carboxylic acid derivatives.

The alkylation according to method 3), where Y¹, Y² or Y³ is CO, CS, SO or SO₂, is conveniently performed by reacting the nitrogen anion of (VI) with (V). The nitrogen anion of (VI) can be prepared in an inert organic solvent, e.g. dimethyl formamide (DMF), dimethylsulfoxide (DMSO) or *N*-methylpyrrolidin-2-one (NMP), by the use of a strong base, e.g. NaH, before the alkylation.

The reduction of the double bond according to method 4) is generally performed by catalytic hydrogenation at low pressure (< 3 atm.) in a Parr apparatus or by using reducing agents such as diborane or hydroboric derivatives as produced *in situ* from NaBH₄ in trifluoroacetic acid in inert solvents such as tetrahydrofuran (THF), dioxane or diethyl ether. Starting materials of formula (VII) may be prepared by methods 1), 3), 7) and 8).

Reduction of amide groups according to methods 5) and 6) is most conveniently performed with lithium aluminium hydride or alane in an inert organic solvent such as e.g. tetrahydrofuran (THF) or diethylether from 0 °C to reflux temperature. Starting materials of formula (VIII) may be prepared by methods 2) whereas starting materials of formula (IX) may be prepared by methods 1), 7) and 8).

The acylation according to method 8) is conveniently performed by the use of coupling reagents such as e.g. dicyclohexyl carbodiimide.

### Experimental Section

Melting points were determined on a Büchi B-540 apparatus and are uncorrected. Mass spectra were obtained on a PE Sciex API 150EX instrument equipped with an APCI source by an alternating scan method to give molecular weight information. The molecular ion, MH+, was obtained at low orifice voltage (5-20V) and fragmentation at high orifice voltage (100-200V). ¹H NMR spectra were recorded at 500.13 MHz on a Bruker Avance DRX500 instrument or at 250.13 MHz on a Bruker AC 250 instrument. Deuterated chloroform (99.8%D) or dimethyl sulfoxide (99.9%D) were used as solvents. TMS was used as internal reference standard. Chemical shift values are expressed in ppm-values. The following abbreviations are used for multiplicity of NMR signals: s=singlet, d=doublet, t=triplet, q=quartet, qui=quintet, h=heptet, dd=double doublet, dt=double triplet, dq=double quartet, tt=triplet of triplets, m=multiplet, b=broad. NMR signals corresponding to acidic protons are generally omitted. Content of water in crystalline compounds was determined by Karl Fischer titration. For column chromatography, silica gel of type Kieselgel 60, 40-60 mesh ASTM was used.

### Examples

### Preparation of intermediates

### A. Alkylating reagents

### 1-(2-Chloroethyl)-3,4-dihydroquinolin-2(1H)-one

A suspension of sodium hydride (3.0 g, 60% in mineral oil) and dimethyl formamide (100 mL) was kept at 15-18 °C followed by the addition of a solution of 3,4-dihydroquinolin-2(1*H*)-one (10.0 g) in dimethyl formamide (150 mL). The resulting mixture was stirred at room temperature for 60 min followed by the addition of a solution of 2-chloroethyl acetate (10.0 g) in dimethyl formamide (50 mL) at a temperature of 20 °C. The resulting mixture was heated at 80 °C for 2 ½ h, cooled and poured onto ice. The aqueous phase was extracted with ethyl acetate, and the combined organic phases were washed with brine, dried (MgSO₄) and concentrated *in vacuo*. The crude product was purified by flash chromatography on silicagel (eluent: ethyl acetate/heptane 1:1) to give crude 1-(2-acetoxyethyl)-3,4-dihydroquinolin-2(1*H*)-one (10.2 g). A mixture of crude 1-(2-acetoxyethyl)-3,4-dihydroquinolin-2(1*H*)-one, sodium methanolate (2.5 mL, 30% in methanol) and methanol (250 mL) was stirred at room temperature for 16 h and subsequently concentrated *in vacuo*. The residue was purified by flash chromatography on silicagel (eluent: ethyl acetate/heptane 1:1) to give the corresponding alcohol as a red crystalline compound (4.9 g). This alcohol was dissolved in tetrahydrofuran (100 mL) followed by the addition of trietylamine (8.2 mL). The resulting mixture was cooled to 5-6 °C followed by the addition of a solution of methane sulfonic acid chloride (2 mL) in tetrahydrofuran (25 mL). The mixture was filtered and evaporated to dryness *in vacuo*. The residue was dissolved in dimethyl formamide (50 mL) followed by addition of lithium chloride (4.9 g), and the resulting mixture was heated at 70 °C for 5 min. The mixture was poured onto brine, and the aqueous phase was extracted with ethyl acetate. The combined organic phases were dried (MgSO₄), filtered and concentrated *in vacuo*. The residue was purified by flash chromatography on silicagel (eluent: ethyl acetate/heptane 1:1) to give the product as a red oil (2.9 g).

### 1-(3-Bromopropan-1-yl)-3,4-dihydroquinolin-2(1H)-one

A suspension of sodium hydride (6.8 g, 60% in mineral oil) and dimethyl formamide (200 mL) was kept at 20-25 °C followed by the addition of a solution of 3,4-dihydroquinolin-2(1*H*)-one (25.0 g) in dimethyl formamide (180 mL). The resulting mixture was stirred at room temperature for 10 min followed by the addition of a solution of 1,3-dibromopropane (172 g) in dimethyl formamide (150 mL) at a temperature of 20-35 °C. The resulting mixture was stirred at 30 °C for 20 min and concentrated *in vacuo*. The residue was poured onto ice, and the aqueous phase was extracted with ethyl acetate. The combined organic phases were washed with brine, dried (MgSO₄) and concentrated *in vacuo*. The crude product was purified by flash chromatography on silicagel (eluent: ethyl acetate/heptane 1:1) to give the product as a yellow oil (27 g).

The following compounds were prepared in a similar manner

### 1-(4-Bromobutan-1-yl)-3,4-dihydroquinolin-2(1H)-one

from 3,4-dihydroquinolin-2(1*H*)-one and 1,4-dibromobutane

### 1-(5-Bromopentan-1-yl)-3,4-dihydroquinolin-2(1H)-one

from 3,4-dihydroquinolin-2(1*H*)-one and 1,5-dibromopentane

### 4-(4-Bromobutan-1-yl)-3,4-dihydro-2H-1,4-benzoxazin-3(4H)-one

from 3,4-dihydro-2*H*-1,4-benzoxazin-3(4*H*)-one and 1,4-dibromobutane

### 2-(3-Hydroxypropan-1-yl)-3,4-dihydroisoquinolin-1(2H)-one

from 3,4-dihydroisoquinolin-1(2*H*)-one and 3-bromopropanol

### 2-(4-Bromobutan-1-yl)-3,4-dihydroisoquinolin-1(2H)-one

from 3,4-dihydroisoquinolin-1(2*H*)-one and 1,4-dibromobutane

### 1-(3-Bromopropan-1-yl)-3,4-dihydroisoquinolin-1(2H)-one

The compound 2-(3-hydroxypropan-1-yl)-3,4-dihydroisoquinolin-1(2*H*)-one was dissolved in tetrahydrofuran (100 mL) followed by the addition of triethylamine (5.2 mL). The resulting mixture was cooled to 6-11 °C followed by the addition of a solution of methane sulfonic acid chloride (1.4 mL) in tetrahydrofuran (25 mL). The mixture was stirred at 5 °C for 10 min, filtered and concentrated *in vacuo*. The residue was dissolved in acetone (250 mL) followed by addition of lithium bromide (6.5 g), and the resulting mixture was boiled under reflux for 2 h. The mixture was poured onto brine, and the aqueous phase was extracted with ethyl acetate. The combined organic phases were dried (MgSO₄), filtered and concentrated *in vacuo*. The residue was purified by flash chromatography on silicagel (eluent: ethyl acetate/heptane 1:2) to give the product as a yellow oil (2.7 g).

### 3-Bromo-1-(3,4-dihydro-1H-isoquinolin-2-yl)propan-1-one

A solution 3,4-dihydro-1*H*-isoquinoline (18.4 g) and triethylamine (19 g) was cooled down to 5 °C followed by the addition of a solution of 3-bromopropanoyl chloride (25 g) in tetrahydrofuran (50 mL). The resulting mixture was stirred at 5 °C for 45 min, filtered and concentrated *in vacuo* and used without any further purification.

The following compound was prepared in a similar manner

### 4-Bromo-1-(3,4-dihydro-1H-isoquinolin-2-yl)butan-1-one

from 3,4-dihydro-1*H*-isoquinoline and 4-bromobutanoyl chloride

### B. Amines

### 5-(Piperazin-1-yl)-1H-indole

A mixture of 5-nitro-1*H*-indole (34 g), palladium on activated carbon (Pd 5 %, water 50 %) (2.5 g) and ethyl acetate was shaken at room temperature for 1.5 h under 3 atmospheres of hydrogen. The mixture was filtered, and the solvent was removed *in vacuo* to yield a crystalline compound (28 g), which was dissolved in tetrahydrofuran (400 mL). The solution was subsequently added to a boiling mixture of *N*-benzyliminodiacetic acid (54.4 g) and 1,1'-carbonyldiimidazole (82.4 g) in tetrahydrofuran (1100 mL), and the resulting mixture was boiled under reflux for 3 h The mixture was filtered and concentrated *in vacuo*. The residue was purified by flash chromatography on silicagel (eluent: ethyl acetate/triethylamine 100:4) to give a white crystalline compound (57.5 g), which subsequently was dissolved in tetrahydrofuran (300 mL) and added to alane in tetrahydrofuran (500 mL) at 5-16 °C. The alane was prepared from lithium aluminium hydride (25 g) and concentrated sulphuric acid (32.3 g). The mixture was stirred at 5 °C for 45 min and subsequently quenched by the addition of water (50 mL), 15 % aqueous sodium hydroxide solution (25 mL) and water (125 mL). The mixture was dried (MgSO₄), filtered and concentrated *in vacuo*. The residue was purified by flash chromatography on silicagel (eluent: ethyl acetate) to give a brown oily compound (44.9 g), which subsequently was dissolved in methanol (1000 mL) and added ammonium formate (150 g) and palladium on activated carbon (Pd 5 %, water 50 %) (12 g). The mixture was boiled under reflux for 45 min, cooled, filtered and concentrated *in vacuo*. The residue was dissolved in tetrahydrofuran/ethyl acetate and added brine and concentrated aqueous ammonia solution under cooling to give a basic reaction mixture. The two phases were separated, and the aqueous phase was extracted an additional two times with tetrahydrofuran/ethyl acetate. The combined organic phases were washed with brine, dried (MgSO₄) and concentrated *in vacuo*. The residue was crystallised from tetrahydrofuran/n-hetane to give the title compound (17.3 g).

### 6-(Piperazin-1-yl)-1H-indole

prepared in a similar manner as 5-(piperazin-1-yl)-1*H*-indole starting from 6-amino-1*H*-indole (Brown et al. *J. Am. Chem. Soc*. **1954,** *76*, 5149-5150)

### 5-(3,6-Dihydro-2H-pyridin-4-yl)-1H-indole

See WO 94/20459.

### 5-(Piperidin-4-yl)-1H-indole

A mixture of 5-(3,6-dihydro-2*H*-pyridin-4-yl)-1*H*-indole (3.4 g), platinum oxide (0.2 g) and acetic acid (50 mL) was shaken at room temperature for 24 h under 3 atmospheres of hydrogen. The mixture was filtered, and the solvent was removed *in vacuo*. The residue was purified by flash chromatography on silicagel (eluent: 4 M ammonia in methanol) to give the title compound (1.3 g).

### Preparation of the compounds of the invention

### Example 1

### 1, 5-{4-[2-(2-Oxo-3,4-dihydro-2H-quinolin-1-yl)ethyl]piperazin-1-yl}-1H-indole, oxalate

A mixture of 5-(piperazin-1-yl)-1*H*-indole (0.5 g), 1-(2-chloroethyl)-3,4-dihydroquinolin-2(1*H*)-one (1.04 g), lithium bromide (1.08 g), triethylamine (1 mL), dimethyl formamide (2 mL), tetrahydrofuran (25 mL) and butanone (25 mL) was boiled under reflux for 5 h. The mixture was filtered and concentrated *in vacuo*. The residue was purified by flash chromatography on silicagel (eluent: ethyl acetate/heptane/triethylamine 50:5:5). The title compound was isolated as the oxalate salt from tetrahydrofuran (0.15 g). ¹H NMR (DMSO-d₆): 2.55 (t, 2H); 2.80-2.95 (m, 4H); 3.05 (s, 4H); 3.15 (s, 4H); 4.15 (t, 2H); 6.30 (s, 1H); 6.85 (d, 1H); 6.95-7.10 (m, 2H); 7.15-7.35 (m, 5H); 10.85 (s, 1H). MS m/z: 375 (MH+), 174.

### Example 2

### 2a, 5-{4-[3-(2-Oxo-3,4-dihydro-2H-quinolin-1-yl)propan-1-yl]piperazin-1-yl}-1H-indole, hydrochloride

A mixture of 5-(piperazin-1-yl)-1*H*-indole (1.8 g) in dimethyl formamide (25 mL) and 1-(3-bromopropan-1-yl)-3,4-dihydroquinolin-2(1*H*)-one (2.9 g) in butanone (100 mL) was added triethylamine (5 mL) and boiled under reflux for 4 h. The mixture was filtered and concentrated *in vacuo*, and the residue was purified by flash chromatography on silicagel (eluent: ethyl acetate/triethylamine 100:4) to give the crude product as an orange oil (3.0 g). The title compound was isolated as the hydrochloride salt from acetone as a white crystalline compound (0.9 g). Mp 246-248 °C. ¹H NMR (DMSO-d₆): 2.00-2.10 (m, 231); 2.55 (t, 2H); 2.90 (t, 2H); 3.25 (broad s, 6H); 3.50-3.70 (m, 4H); 3.95 (t, 2H); 6.35 (s, 1H); 6.95 (d, 1H); 7.05 (t, 1H); 7.15-7.40 (m, 6H); 11.05 (broad s, 2H). MS m/z: 389 (MH+), 188.

The following compounds were prepared in a similar manner:

### 2b, 5-{4-[4-(2-Oxo-3,4-dihydro-2H-quinolin-1-yl)butan-1-yl]piperazin-1-yl}-1H-indole, hydrochloride

from 5-(piperazin-1-yl)-1*H*-indole and 1-(4-bromobutan-1-yl)-3,4-dihydroquinolin-2(1*H*)-one. Mp 233-236 °C. ¹H NMR (DMSO-d₆): 1.55-1.65 (m, 2H); 1.75-1.85 (m, 2H); 2.55 (t, 2H); 2.85 (t, 2H); 3.20 (s, 2H); 3.35 (broad s, 2H); 3.45 (broad s, 2H); 3.55-3.75 (m, 4H); 3.95 (t, 2H); 6.40 (s, 1H); 7.00 (t, 1H); 7.05 (d, 1H); 7.15 (d, 1H); 7.20-7.30 (m, 3H); 7.35 (s, 1H); 7.40 (d, 1H); 11.15 (broad s, 2H). MS m/z: 403 (MH+).

### 2c, 6-{4-[3-(2-Oxo-3,4-dihydro-2H-quinolin-1-yl)propan-1-yl]piperazin-1-yl}-1H-indole, oxalate

from 6-(piperazin-1-yl)-1*H*-indole and 1-(3-bromopropan-1-yl)-3,4-dihydroquinolin-2(1*H*)-one. ¹H NMR (DMSO-d₆): 1.80-1.95 (m, 2H); 2.55 (t, 2H); 2.75-2.90 (m, 4H); 3.00 (s, 4H); 3.20 (s, 4H); 3.95 (t, 2H); 6.30 (s, 1H); 6.80 (d, 1H); 6.85 (s, 1H); 7.00 (t, 1H); 7.10-7.30 (m, 5H); 7.40 (d, 1H); 10.80 (s, 1H). MS m/z: 389 (MH+), 188.

### 2d, 6-{4-[4-(2-Oxo-3,4-dihydro-2H-quinolin-1-yl)butan-1-yl]piperazin-1-yl}-1H-indole, oxalate

from 6-(piperazin-1-yl)-1*H*-indole and 1-(4-bromobutan-1-yl)-3,4-dihydroquinolin-2(1*H*)-one: ¹H NMR (DMSO-d₆): 1.55-1.75 (m, 4H); 2.55 (t, 2H); 2.85 (t, 2H); 2.95 (t, 2H); 3.15 (s, 4H); 3.25 (s, 4H); 3.95 (t, 2H); 6.30 (s, 1H); 6.80 (d, 1H); 6.90 (s, 1H); 7.00 (t, 1H); 7.10-7.30 (m, 4H); 7.40 (d, 1H); 10.80 (s, 1H). MS m/z: 403 (MH+).

### 2e, 5-{1-[3-(2-Oxo-3,4-dihydro-2H-quinolin-1-yl)propan-1-yl]-3,6-dihydro-2H-pyridin-4-yl}-1H-indole, hydrochloride

from 5-(3,6-dihydro-2*H*-pyridin-4-yl)-1*H*-indole and 1-(3-bromopropan-1-yl)-3,4-dihydroquinolin-2(1*H*)-one. ¹H NMR (DMSO-d₆): 2.00-2.15 (m, 2H); 2.55 (t, 2H); 2.75-2.95 (m, 4H); 3.25 (s, 4H); 3.55-3.80 (m, 2H); 3.90-4.10 (m, 2H); 6.05 (s, 1H); 6.45 (s, 1H); 7.05 (t, 1H); 7.15-7.30 (m, 4H); 7.30-7.45 (m, 2H); 7.65 (s, 1H); 10.30 (b s, 1H); 11.20 (s, 1H). MS m/z: 386 (MH+) 217,149.

### 2f, 5-{1-[3-(2-Oxo-3,4-dihydro-2H-quinolin-1-yl)propan-1-yl]piperidin-4-yl}-1H-indole, hydrochloride

from 5-(piperidin-4-yl)-1*H*-indole and 1-(3-bromopropan-1-yl)-3,4-dihydroquinolin-2(1*H*)-one. ¹H NMR (DMSO-d₆): 1.85-2.05 (m, 6H); 2.55-2.60 (m, 2H); 2.80-2.95 (m, 3H); 3.00-3.15 (m, 2H); 3.15-3.20 (m, 2H); 3.55 (d, 2H); 3.95 (t, 2H); 6.35 (s, 1H); 6.95 (d, 1H); 7.05 (t, 1H); 7.15-7.40 (m, 6H); 9.45 (b s, 1H); 11.00 (s, 1H). MS m/z: 388 (MH+) 188.

### Example 3

### 3a, 5-{4-[4-(3-Oxo-3,4-dihydro-2H-1,4-benzoxazin-4-yl)butan-1-yl]piperazin-1-yl}-1H-indole, hydrochloride

A mixture of 5-(piperazin-1-yl)-1*H*-indole (0.3 g), 4-(4-bromobutan-1-yl)-3,4-dihydro-2*H*-1,4-benzoxazin-3(4*H*)-one (0.55 g) and triethylamine (0.75 g) in dimethyl formamide (5 mL) and butanone (10 mL) was boiled under reflux for 6 h. The mixture was concentrated *in vacuo*, and the residue was purified by flash chromatography on silicagel (eluent: ethyl acetate/ethanol/triethylamine 90:10:5) to give the crude product, which was isolated as the hydrochloride salt from tetrahydrofuran as a white crystalline compound (0.3 g). ¹H NMR (DMSO-d₆): 1.55-1.70 (m, 2H); 1.80-1.90 (m, 2H); 3.20 (t, 2H); 3.40 (broad s, 2H); 3.50-3.80 (m, 6H); 3.95 (t, 2H); 4.65 (s, 2H); 6.40 (s, 1H); 6.95-7.15 (m, 4H); 7.25 (d, 1H); 7.40 (s, 1H); 7.45 (d, 1H); 11.20 (s, 1H); 11.40 (broad s). MS m/z: 405 (MH+). The following compounds were prepared in a similar manner

### 3b, 5-{4-[3-(1-Oxo-3,4-dihydro-1H-quinolin-2-yl)propan-1-yl]piperazin-1-yl}-1H-indole, hydrochloride

from 5-(piperazin-1-yl)-1*H*-indole and 1-(3-bromopropan-1-yl)-3,4-dihydroisoquinolin-1(2*H*)-one. ¹H NMR (DMSO-d₆): 2.05-2.20 (m, 2H); 3.05 (t, 2H); 3.20 (t, 2H); 3.30-3.80 (m, 12H); 6.40 (s, 1H); 7.05 (d, 1H); 7.25-7.45 (m, 5H); 7.50 (t, 1H); 7.90 (d, 1H); 11.10 (s, 1H); 11.30 (broad s, 1H). MS m/z: 389 (MH+), 188.

### 3c, 5-{4-[4-(1-Oxo-3,4-dihydro-1H-quinolin-2-yl)butan-1-yl]piperazin-1-yl}-1H-indole, hydrochloride

from 5-(piperazin-1-yl)-1*H*-indole and 2-(4-bromobutan-1-yl)-3,4-dihydroisoquinolin-1(2*H*)-one. ¹H NMR (DMSO-d₆): 1.60-1.70 (m, 2H); 1.70-1.90 (m, 2H); 3.00 (t, 2H); 3.15-3.75 (m, 14H); 6.40 (s, 1H); 7.05 (d, 1H); 7.25-7.40 (m, 5H); 7.45 (t, 1H); 7.90 (d, 1H); 11.10 (s, 1H); 11.25 (broad s, 1H). MS m/z: 403 (MH+).

### Example 4

### 4a, 5-{4-[3-(3,4-Dihydro-2H-quinolin-1-yl)propan-1-yl]piperazin-1-yl}-1H-indole, hydrochloride

A suspension of lithium aluminium hydride (0.56 g) in tetrahydrofuran (50 mL) was stirred at 5 °C followed by the addition of concentrated sulfuric acid (0.73 g) in tetrahydroforan (25 mL). The mixture was stirred at 5 °C for 30 min followed by the addition of 5-{4-[3-(2-oxo-3,4-dihydro-2*H*-quinolin-1-yl)propan-1-yl]piperazin-1-yl)-1*H*-indole (1.8 g) in tetrahydrofuran (50 mL). The mixture was stirred at 5 °C for 15 min followed by standard work up. The title compound was isolated as the hydrochloride salt from acetone as a white crystalline compound (1.4 g). Mp 200-204 °C. ¹H NMR (DMSO-d₆): 1.80-1.95 (m, 2H); 1.95-2.10 (m, 2H); 2.65-2.75 (m, 2H); 3.10-3.80 (m, 14H); 6.35 (s, 1H); 6.50 (t, 1H); 6.65 (d, 1H); 6.90 (d, 1H); 6.90-7.00 (m, 2H); 7.25 (s, 1H); 7.30-7.40 (m, 2H); 11.05 (broad s, 2H). MS m/z: 375 (MH+), 199.

The following compound was prepared in a similar manner

### 4b, 5-{4-[3-(3,4-Dihydro-2H-quinolin-1-yl)propan-1-yl]piperazin-1-yl}-1H-indole, hydrochloride

from 5-{4-[4-(2-oxo-3,4-dihydro-2*H*-quinolin-1-yl)butan-1-yl]piperazin-1-yl}-1*H*-indole. Mp 206-209 °C. ¹H NMR (DMSO-d₆): 1.65-1.80 (m, 2H); 1.80-1.90 (m, 2H); 1.90-2.05 (m, 2H); 2.75-2.85 (m, 2H); 2.25 (t, 2H); 3.40 (s, 4H); 3.60 (broad s, 2H); 3.70-3.90 (m, 4H); 4.00 (broad s, 2H); 6.55 (s, 1H); 6.85 (broad s, 1H); 7.00-7.20 (m, 3H); 7.35 (d, 1H); 7.50 (s, 1H); 7.55 (d, 1H); 7.80 (s, 1H); 11.45 (s, 1H); 12.00 (broad s, 1H). MS m/z: 389 (MH+), 256.

### Example 5

### 5a, 5-{4-[3-(3,4-Dihydro-1H-isoquinolin-2-yl)-3-oxopropan-1-yl]piperazin-1-yl}-1H-indole, hydrochloride

A mixture of 5-(piperazin-1-yl)-1*H*-indole (0.6 g), 3-bromo-1-(3,4-dlydro-1*H*-isoquinolin-2-yl)propan-1-one (1.2 g) and potassium carbonate (0.41 g) was heated at 80 °C for 16 h. The mixture was concentrated *in vacuo*, and the residue was purified by flash chromatography on silicagel (eluent: ethyl acetate/ethanol/triethylamine 90:5:5) to give the crude product, which was isolated as the hydrochloride salt from tetrahydrofuran as a white crystalline compound. Mp 209-211 °C. ¹H NMR (DMSO-d₆): 2.75-2.85 (m, 0.8H); 2.85-3.00 (m, 1.2H); 3.15 (t, 2H); 3.50 (t, 2H); 3.65 (s, 2H); 3.75 (t, 2H); 3.85 (s, 6H); 4.65 (s, 1.2H); 4.75 (s, 0.8H); 6.50 (s, 1H); 7.15-7.25 (m, 4H); 7.30 (d, 1H); 7.45 (s, 1H); 7.55 (d, 1H); 7.70 (s, 1H); 11.40 (s, 1H). MS m/z: 389 (MH+), 252, 214.

The following compound was prepared in a similar manner

### 5b, 5-{4-[4-(3,4-Dihydro-1H-isoquinolin-2-yl)-4-oxobutan-1-yl]piperazin-1-yl}-1H-indole

from 5-(piperazin-1-yl)-1*H*-indole and 4-bromo-1-(3,4-dihydro-1*H*-isoquinolin-2-yl)butan-1-one). Mp 122-125 °C. ¹H NMR (DMSO-d₆): 1.70-1.85 (m, 2H); 2.30-2.40 (m, 2H); 2.40-2.50 (m, 2H); 2.75-2.80 (m, 0.8H); 2.85-2.90 (m, 1.2H); 3.05 (s, 4H); 3.35 (s, 4H); 3.70 (t, 2H); 4.60 (s, 1.2H); 4.70 (s, 0.8H); 6.30 (s, 1H); 6.35 (d, 1H); 7.00 (s, 1H); 7.15-7.35 (m, 6H); 10.80 (s, 1H). MS m/z: 403 (MH+), 270.

### Pharmacological Testing

The compounds of the invention were tested in well- recognised and reliable tests. The tests were as follows:

### Inhibition of the binding of [³H]YM-09151-2 to human dopamine D_{4.2} receptors

By this method, the inhibition by drugs of the binding of [³H]YM-09151-2 (0.06 nM) to membranes of human cloned dopamine D_{4.2} receptors expressed in CHO-cells is determined *in vitro*. Method modified from NEN Life Science Products, Inc., technical data certificate PC2533-10/96.

### Inhibition of the binding of [³H]Ketanserin to 5-HT_{2A} receptors

The compounds were tested with respect to their affinity for 5-HT_{2A} receptors by determining their ability to inhibit the binding of [³H]Ketanserin (0.50 nM) to membranes from rat brain (cortex) *in vitro*. Method described in Sánchez et al. *Drug Dev. Res*. **1991,** *22*, 239-250.

The results obtained are presented in table 1:

**Table 1:**

| Binding Data (IC₅₀ values in nM); or % inhibition. | | |
|---|---|---|
| **Comp.** **No.** | **D**_{**4**}**-bind.** | **5-HT**_{**2A**}**-bind.** |
| **1** | 90 % at 50 nM | 29 |
| **2a** | 3.2 | 13 |
| **2b** | 18 | 17 |
| **2c** | 94 % at 50 nM | 32 |
| **2d** | 58 % at 50 nM | 46 % at 100 nM |
| **2e** | 0.97 | 6.6 |
| **2f** | 1.6 | 22 |
| **3a** | 56 % at 50 nM | 89 % at 100 nM |
| **3b** | 78 % at 50 nM | 90 |
| **3c** | 56 % at 50 nM | 93 % at 100 nM |
| **4a** | 2.6 | 27 |
| **4b** | 8.8 | 19 |
| **5a** | 73 % at 50 nM | 29 % at 100 nM |
| **5b** | 81 % at 50 nM | 4.0 |

In general, the compounds of the invention have been found potently to inhibit the binding of [³H]YM-09151-2 to dopamine D₄ receptors. The compounds have also been tested in a functional assay described by Gazi et al. in *Br. J. Pharmcol*, **1999,** *128*, 613-620, and it has been shown that the compounds are antagonists or partial agonists at dopamine D₄ receptors.

The compounds have also been found to inhibit the binding of [³H]Ketanserin to 5-HT_{2A} receptors *in vitro*.

In addition to the above tests, the compounds of the invention were tested in the following test:

### Inhibition of binding of [³H]Spiperone to rat dopamine D₂ receptors

By this method, the inhibition of drugs of the binding of [³H]Spiperone (0.5 nM) to dopamine D₂ receptors in membranes from rat corpus striatum is determined *in vitro*. Method and results in Hyttel et al. *J. Neurochem*. **1985**, *44*, 1615-1622.

### Inhibition of binding of [³H]Prazosine to rat alpha-1-receptors

By this method, the inhibition by drugs of the binding of [³H]Prazosin (0.25 nM) to alpha-1 receptors in membranes from rat brain is determined *in vitro.* Method modified from Hyttel et al. *J. Neurochem*. **1985,** *44*, 1615-1622.

The compounds have no or only low affinity for the dopamine D₂ receptor and some of the compounds have very low affinity to alpha-1 adrenergic receptors.

Furthermore, some of the compounds also have advantageous good solubility and metabolic stability.

Accordingly, the compounds of the invention are considered useful in the treatment of positive and negative symptoms of schizophrenia, other psychoses, anxiety disorders, such as generalised anxiety disorder, panic disorder and obsessive compulsive disorder, depression, aggression, cognitive disorders side effects induced by conventional antipsychotic agents, migraine, attention deficit hyperactivity disorder and in the improvement of sleep. In particular the compounds of the invention are considered useful in the treatment of positive and negative symptoms of schizophrenia without inducing extrapyramidal side effects.

### Formulation Examples

The pharmaceutical formulations of the invention may be prepared by conventional methods in the art.

For example: Tablets may be prepared by mixing the active ingredient with ordinary adjuvants and/or diluents and subsequently compressing the mixture in a conventional tabletting machine. Examples of adjuvants or diluents comprise: corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.
Solutions for injections may be prepared by dissolving the active ingredient and possible additives in a part of the solvent for injection, preferably sterile water, adjusting the solution to desired volume, sterilising the solution and filling it in suitable ampules or vials. Any suitable additive conventionally used in the art may be added, such as tonicity agents, preservatives, antioxidants, etc.

Typical examples of recipes for the formulation of the compounds of the invention are as follows:
1) Tablets containing 5.0 mg of the active compound calculated as the five base:

| | |
|---|---|
| Active compound | 5.0 mg |
| Lactose | 60 mg |
| Maize starch | 30 mg |
| Hydroxypropylcellulose | 2.4 mg |
| Microcrystalline cellulose | 19.2 mg |
| Croscannellose Sodium Type A | 2.4 mg |
| Magnesium stearate | 0.84 mg |

2) Tablets containing 0.5 mg of the active compound calculated as the free base:

| | |
|---|---|
| Active compound | 0.5 mg |
| Lactose | 46.9 mg |
| Maize starch | 23.5 mg |
| Povidone | 1.8 mg |
| Microcrystalline cellulose | 14.4 mg |
| Croscarmellose Sodium Type A | 1.8 mg |
| Magnesium stearate | 0.63 mg |

3) Syrup containing per millilitre:

| | |
|---|---|
| Active compound | 25 mg |
| Sorbitol | 500 mg |
| Hydroxypropylcellulose | 15 mg |
| Glycerol | 50 mg |
| Methyl-paraben | 1 mg |
| Propyl-paraben | 0.1 mg |
| Ethanol | 0.005 ml |
| Flavour | 0.05 mg |
| Saccharin sodium | 0.5 mg |
| Water | ad 1 ml |

4) Solution for injection containing per millilitre:

| | |
|---|---|
| Active compound | 0.5 mg |
| Sorbitol | 5.1 mg |
| Acetic Acid | 0.05 mg |
| Saccharin sodium | 0.5 mg |
| Water | ad 1 ml |

## Claims

1. An indole derivative having the formula 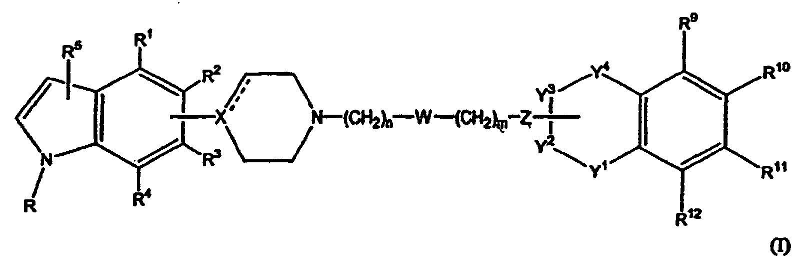 wherein
a) Y¹ is N, which is bound to Z, Z and Y² are selected from CH₂, CO, CS, SO and SO₂; provided that at least one of Z and Y² is CH₂; Y³ is O, S or CHR⁷ and Y⁴ is O, S or CHR⁸, provided that only one of Y³ and Y⁴ is O or S;
b) Y² is N, which is bound to Z, Z and Y¹ are selected from CH₂, CO, CS, SO and SO₂; provided that at least one of Z and Y¹ is CH₂; Y³ is CHR⁷ and Y⁴ is O, S or CHR⁸;
c) Y² is N, which is bound to Z, Z and Y³ are selected from CH₂, CO, CS, SO and SO₂ provided that at least one of Z and Y³ is CH₂; Y¹ is CHR⁶ and Y⁴ is O, S or CHR⁸;
W is a bond, O, S, CO, CS, SO or SO₂;
X is C, CH or N, provided that when X is C, the dotted line indicates a bond, and when X is N or CH, the dotted line indicates no bond;
n is 0-5, m is 0-5 and n + m is 1-6; provided that when W is O or S, then n ≥ 2 and m ≥ 1; when W is CO, CS, SO or SO₂, then n ≥ 1and m ≥ 1;
one of R¹, R², R³ and R⁴ forms a bond to X and the others of R¹, R², R³,R⁴ and R⁵ and R⁹-R¹² are independently selected from hydrogen, halogen, cyano, nitro, amino, hydroxy, C₁₋₆-alkyl-amino, di-( C₁₋₆-alkyl)-amino, C₁₋₆-alkyl, C₂₋₆-alkenyl, C₂₋₆-alkynyl, C₁₋₆ alkoxy, C₁₋₆-alkylthio, C₁₋₆-alkyl substituted with hydroxy or thiol, C₃₋₈-cycloalkyl, C₃₋₈-cycloalkyl-C₁₋₆-alkyl, acyl, thioacyl, trifluoromethyl, trifluoromethylsulfonyl or C₁₋₆ alkylsulfonyl;
R⁶, R⁷ and R⁸ are hydrogen or halogen;
R is hydrogen, C₁₋₆-alkyl, C₂₋₆-alkenyl, C₂₋₆-alkynyl, C₁₋₆-alkyl substituted with hydroxy or thiol, C₃₋₈-cycloalkyl, C₃₋₈-cycloalkyl-C₁₋₆-alkyl, acyl, thioacyl, trifluoromethylsulfonyl and C₁₋₆ alkylsulfonyl;
or pharmaceutically acceptable salts thereof.

2. A compound according to claim 1, wherein Y¹ is N, which is bound to Z, Z and Y² are selected from CH₂, CO, CS, SO and SO₂; provided that at least one of Z and Y² is CH₂; Y³ is O, S or CHR⁷ and Y⁴ is O, S or CHR⁸, provided only one of Y³ and Y⁴ is O or S;

3. A compound according to claim 2, wherein Y¹ is N, which is bound to Z, Z and Y² are selected from CH₂ and CO; provided that at least one of Z and Y² is CH₂; Y³ is CHR⁷ and Y⁴ is O, S or CHR⁸.

4. A compound according to claim 2, wherein Y¹ is N, which is bound to Z, Z is CH₂, Y² is CO, Y³ is CHR⁷ and Y⁴ is O, S or CHR⁸.

5. A compound according to claim 2 wherein Y¹ is N, which is bound to Z, Z is CH₂, Y² is CO, Y³ is CH₂ and Y⁴ is CH₂.

6. A compound according to claim 2, wherein Y¹ is N, which is bound to Z, Z and Y² are CH₂; Y³ is CHR⁷ and Y⁴ is O, S or CHR⁸.

7. A compound according to claim 2, wherein Y¹ is N, which is bound to Z, Z is CO, Y² is CH₂, Y³ is CHR⁷ and Y⁴ is O, S or CHR⁸.

8. A compound according to claim 1, wherein Y² is N, which is bound to Z, Z and Y¹ are selected from CH₂, CO, CS, SO and SO₂; provided that at least one of Z and Y¹ is CH₂; Y³ is CHR⁷ and Y⁴ is O, S, or CHR⁸.

9. A compound according to claim 8, wherein Y² is N, which is bound to Z, Z and Y¹ are selected from CH₂ and CO; provided that at least one of Z and Y¹ is CH₂; Y³ is CHR⁷ and Y⁴ is O, S or CHR⁸.

10. A compound according to claim 8, wherein Y² is N, which is bound to Z, Z is CH₂ and Y¹ is CO; Y³ is CHR⁷ and Y⁴ is O, S or CHR⁸.

11. A compound according to claim 8, wherein Y² is N, which is bound to Z, Z is CH₂ and Y¹ is CO; Y³ is CH₂ and Y⁴ is CH₂.

12. A compound according to claim 8, wherein Y² is N, which is bound to Z, Z and Y¹ are CH₂; Y³ is CHR⁷ and Y⁴ is O, S or CHR⁸.

13. A compound according to claim 8, wherein Y² is N, which is bound to Z, Z is CO and Y¹ is CH₂; Y³ is CHR⁷ and Y⁴ is O, S or CHR⁸.

14. A compound according to claim 8, wherein Y² is N, which is bound to Z, Z is CO and Y¹ is CH₂; Y³ is CH₂ and Y⁴ is CH₂.

15. A compound according to claim 1, wherein Y² is N, which is bound to Z, Z and Y³ are selected from CH₂, CO, CS, SO and SO₂ provided that at least one of Z and Y³ is CH₂; Y¹ is CHR⁶ and Y⁴ is O, S or CHR⁸,

16. A compound according to claim 15, wherein Y² is N, which is bound to Z, Z and Y³ are selected from CH₂ and CO; Y¹ is CHR⁶ and Y⁴ is O S or CHR⁸.

17. A compound according to claim 15, wherein Y² is N, which is bound to Z, Z is CH₂ and Y³ is CO; Y¹ is CHR⁶ and Y⁴ is O, S or CHR⁸.

18. A compound according to claim 15, wherein Y² is N, which is bound to Z, Z and Y³ are CH₂; Y¹ is CHR⁶ and Y⁴ is O, S or CHR⁸.

19. A compound according to claim 15, wherein Y² is N, which is bound to Z, Z is CO and Y³ is CH₂; Y¹ is CHR⁶ and Y⁴ is O, S or CHR⁸.

20. A compound according to claims 1-19, wherein one of R¹, R², R³ and R⁴ forms a bond to X and the others of R¹, R², R³, R⁴ and R⁵ and R⁹-R¹² are selected from hydrogen, halogen, cyano, nitro, amino, C₁₋₆-alkyl, C₁₋₆ alkoxy, C₁₋₆-alkylthio, hydroxy and trifluoromethyl, and R is hydrogen, C₁₋₆-alkyl, C₁₋₆-alkylcarbonyl.

21. A compound according to claim 20, wherein R² or R³ form a bond to X.

22. A compound according to claims 1-19, wherein X is N.

23. A compound according to claims 1-19, wherein X is C.

24. A compound according to claims 1-19, wherin X is CH.

25. A compound according to claims 1-19, wherein R⁶, R⁷ and R⁸ are hydrogen.

26. A compound according to claims 1-19, wherein R, R¹-R⁵, and R⁹-R¹² are hydrogen or halogen.

27. A compound according to claims 1-19, wherein W is a bond and n + m is 2 to 4.

28. A compound according to claim 1 which is selected from
5-{4-[2-(2-Oxo-3,4-dihydro-2*H*-quinolin-1-yl)ethyl]piperazin-1-yl}-1*H*-indole,
5-{4-[3-(2-Oxo-3,4-dihydro-2*H*-quinolin-1-yl)propan-1-yl]piperazin-1-yl}-1*H*-indole,
5-{4-[4-(2-Oxo-3,4-dihydro-2*H*-quinolin-1-yl)butan-1-yl]piperazin-1-yl}-1*H*-indole,
6-{4-[3-(2-Oxo-3,4-dihydro-2*H*-quinolin-1-yl)propan-1-yl]piperazin-1-yl}-1*H*-indole,
6-{4-[4-(2-Oxo-3,4-dihydro-2*H*-quinolin-1-yl)butan-1-yl]piperazin-1-yl}-1*H*-indole,
5-{4-[3-(2-Oxo-3,4-dihydro-2*H*-quinolin-1-yl)propan-1-yl]-3,6-dihydro-2*H*-pyridin-4-yl}-1H-indole,
5-{4-[3-(2-Oxo-3,4-dihydro-2*H*-quinolin-1-yl)propan-1-yl]piperidin-4-yl}-1*H*-indole,
5-{4-[4-(3-Oxo-3,4-dihydro-2*H*-1,4-benzoxazin-4-yl)butan-1-yl]piperazin-1-yl}-1*H*-indole,
5-{4-[3-(1-Oxo-3,4-dihydro-1*H*-quinolin-2-yl)propan-1-yl]piperazin-1-yl}-1*H*-indole,
5-{4-[4-(1-Oxo-3,4-dihydro-1*H*-quinolin-2-yl)butan-1-yl]piperazin-1-yl}-1*H*-indole,
5-{4-[3-(3,4-Dihydro-2*H*-quinolin-1-yl)propan-1-yl]piperazin-1-yl}-1*H*-indole,
5-{4-[3-(3,4-Dihydro-2*H*-quinolin-1-yl)propan-1-yl]piperazin-1-yl}-1*H*-indole,
5-{4-[3-(3,4-dihydro-1*H*-isoquinolin-2-yl)-3-oxopropan-1-yl]piperazin-1-yl}-1*H*-indole, and
5-{4-[4-(3,4-dihydro-1*H*-isoquinolin-2-yl)-4-oxobutan-1-yl]piperazin-1-yl}-1*H*-indole, or pharmaceutically acceptable salts thereof.

29. A pharmaceutical composition **characterised in that** it comprises a compound of any of claims 1 to 28 in a therapeutically effective amount together with one or more pharmaceutically acceptable carriers or diluents.

30. Use of a compound of any of Claims 1 to 28 for the manufacture of a medicament useful in the treatment of positive and negative symptoms of schizophrenia, other psychoses, anxiety disorders, such as generalised anxiety disorder, panic disorder and obsessive compulsive disorder, depression, aggression, cognitive disorders, side effects induced by conventional antipsychotic agents, migraine, attention deficit hyperactivity disorder and in the improvement of sleep.

31. A compound of any of claims 1 to 28 for use in a method of treating the positive and negative symptoms of schizophrenia, other psychoses, anxiety disorders, such as generalised anxiety disorder, panic disorder, and obsessive compulsive disorder, depression, aggression, cognitive disorders, side effects induced by conventional antipsychotic agents, migraine, attention deficit hyperactivity disorder and in the improvement of sleep comprising administration of a therapeutically acceptable amount of a compound according to any of claims 1 to 28.

## Patentansprüche

1. Indol-Derivat mit der Formel 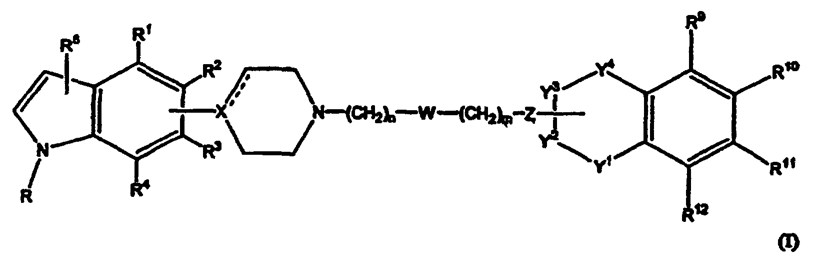 worin
a) Y¹ N ist, das an Z gebunden ist, Z und Y² aus CH₂, CO, CS, SO und SO₂ ausgewählt sind; mit der Maßgabe, daß wenigstens eines aus Z und Y² CH₂ ist; Y³ O, S oder CHR⁷ ist und Y⁴ O, S oder CHR⁸ ist, mit der Maßgabe, daß nur eines aus Y³ und Y⁴ O oder S ist;
b) Y² N ist, das an Z gebunden ist, Z und Y¹ aus CH₂, CO, CS, SO und SO₂ ausgewählt sind; mit der Maßgabe, das wenigstens eines aus Z und Y¹ CH₂ ist; Y³ CHR⁷ ist und Y⁴ O, S oder CHR⁸ ist;
c) Y² N ist, das an Z gebunden ist, Z und Y³ aus CH₂, CO, CS, SO und SO₂ ausgewählt sind, mit der Maßgabe, daß wenigstens eines aus Z und Y³ CH₂ ist; Y¹ CHR⁶ ist und Y⁴ O, S oder CHR⁸ ist;
W eine Bindung, O, S, CO, CS, SO oder SO₂ ist;
X C, CH oder N ist, mit der Maßgabe, daß dann, wenn X C ist, die gestrichelte Linie eine Bindung anzeigt, und daß dann, wenn X N oder CH ist, die gestrichelte Linie keine Bindung anzeigt;
n 0-5 ist, m 0-5 ist und n + m 1-6 ist; mit der Maßgabe, daß dann, wenn W 0 oder S ist, n ≥ 2 und m ≥ 1 ist; und daß dann, wenn W CO, CS, SO oder SO₂ ist, n ≥ 1 und m ≥ 1 ist;
eines aus R¹, R², R³ und R⁴ eine Bindung mit X bildet und die anderen aus R¹, R², R³, R⁴ und R⁵ und R⁹-R¹² unabhängig ausgewählt sind aus Wasserstoff, Halogen, Cyano, Nitro, Amino, Hydroxy, C₁₋₆-Alkylamino, Di(C₁₋₆-alkyl)-amino, C₁₋₆-Alkyl, C₂₋₆-Alkenyl, C₂₋₆-Alkinyl, C₁₋₆-Alkoxy, C₁₋₆-Alkylthio, C₁₋₆-Alkyl, das mit Hydroxy oder Thiol substituiert ist, C₃₋₈-Cycloalkyl, C₃₋₈-Cycloalkyl-C₁₋₆-alkyl, Acyl, Thioacyl, Trifluormethyl, Trifluormethylsulfonyl oder C₁₋₆-Alkylsulfonyl;
R⁶, R⁷ und R⁸ Wasserstoff oder Halogen sind;
R Wasserstoff, C₁₋₆-Alkyl, C₂₋₆-Alkenyl, C₂₋₆-Alkinyl, C₁₋₆-Alkyl, das mit Hydroxy oder Thiol substituiert ist, C₃₋₈-Cycloalkyl, C₃₋₈-Cycloalkyl-C₁₋₆-alkyl, Acyl, Thioacyl, Trifluormethylsulfonyl und C₁₋₆-Alkylsulfonyl ist;
oder pharmazeutisch akzeptable Salze davon.

2. Verbindung gemäß Anspruch 1, worin Y¹ N ist, das an Z gebunden ist, Z und Y² aus CH₂, CO, CS, SO und SO₂ ausgewählt sind; mit der Maßgabe, daß wenigstens eines aus Z und Y² CH₂ ist; Y³ O, S oder CHR⁷ ist und Y⁴ O, S oder CHR⁸ ist, mit der Maßgabe, daß nur eines aus Y³ und Y⁴ O oder S ist.

3. Verbindung gemäß Anspruch 2, worin Y¹ N ist, das an Z gebunden ist, Z und Y² aus CH₂ und CO ausgewählt sind; mit der Maßgabe, daß wenigstens eines aus Z und Y² CH₂ ist; Y³ CHR⁷ ist und Y⁴ O, S oder CHR⁸ ist.

4. Verbindung gemäß Anspruch 2, worin Y¹ N ist, das an Z gebunden ist, Z CH₂ ist, Y² CO ist, Y³ CHR⁷ ist und Y⁴ O, S oder CHR⁸ ist.

5. Verbindung gemäß Anspruch 2, worin Y¹ N ist, das an Z gebunden ist, Z CH₂ ist, Y² CO ist, Y³ CH₂ ist und Y⁴ CH₂ ist.

6. Verbindung gemäß Anspruch 2, worin Y¹ N ist, das an Z gebunden ist, Z und Y² CH₂ sind; Y³ CHR⁷ ist und Y⁴ O, S oder CHR⁸ ist.

7. Verbindung gemäß Anspruch 2, worin Y¹ N ist, das an Z gebunden ist, Z CO ist, Y² CH₂ ist, Y³ CHR⁷ ist und Y⁴ O, S oder CHR⁸ ist.

8. Verbindung gemäß Anspruch 1, worin Y² N ist, das an Z gebunden ist, Z und Y¹ aus CH₂, CO, CS, SO und SO₂ ausgewählt sind; mit der Maßgabe, daß wenigstens eines aus Z und Y¹ CH₂ ist; Y³ CHR⁷ ist und Y⁴ O, S oder CHR⁸ ist.

9. Verbindung gemäß Anspruch 8, worin Y² N ist, das an Z gebunden ist, Z und Y¹ aus CH₂ und CO ausgewählt sind; mit der Maßgabe, daß wenigstens eines aus Z und Y¹ CH₂ ist; Y³ CHR⁷ ist und Y⁴ O, S oder CHR⁸ ist.

10. Verbindung gemäß Anspruch 8, worin Y² N ist, das an Z gebunden ist, Z CH₂ ist und Y¹ CO ist; Y³ CHR⁷ ist und Y⁴ O, S oder CHR⁸ ist.

11. Verbindung gemäß Anspruch 8, worin Y² N ist, das an Z gebunden ist, Z CH₂ ist und Y¹ CO ist; Y³ CH₂ ist und Y⁴ CH₂ ist.

12. Verbindung gemäß Anspruch 8, worin Y² N ist, das an Z gebunden ist, Z und Y¹ CH₂ sind; Y³ CHR⁷ ist und Y⁴ O, S oder CHR⁸ ist.

13. Verbindung gemäß Anspruch 8, worin Y² N ist, das an Z gebunden ist, Z CO ist und Y¹ CH₂ ist; Y³ CHR⁷ ist und Y⁴ O, S oder CHR⁸ ist.

14. Verbindung gemäß Anspruch 8, worin Y² N ist, das an Z gebunden ist, Z CO ist und Y¹ CH₂ ist; Y³ CH₂ ist und Y⁴ CH₂ ist.

15. Verbindung gemäß Anspruch 1, worin Y² N ist, das an Z gebunden ist, Z und Y³ aus CH₂, CO, CS, SO und SO₂ ausgewählt sind, mit der Maßgabe, daß wenigstens eines aus Z und Y³ CH₂ ist; Y¹ CHR⁶ ist und Y⁴ O, S oder CHR⁸ ist.

16. Verbindung gemäß Anspruch 15, worin Y² N ist, das an Z gebunden ist, Z und Y³ aus CH₂ und CO ausgewählt sind; Y¹ CHR⁶ ist und Y⁴ O, S oder CHR⁸ ist.

17. Verbindung gemäß Anspruch 15, worin Y² N ist, das an Z gebunden ist, Z CH₂ ist und Y³ CO ist; Y¹ CHR⁶ ist und Y⁴ O, S oder CHR⁸ ist.

18. Verbindung gemäß Anspruch 15, worin Y² N ist, das an Z gebunden ist, Z und Y³ CH₂ sind; Y¹ CHR⁶ ist und Y⁴ O, S oder CHR⁸ ist.

19. Verbindung gemäß Anspruch 15, worin Y² N ist, das an Z gebunden ist, Z CO ist und Y³ CH₂ ist; Y¹ CHR⁶ ist und Y⁴ O, S oder CHR⁸ ist.

20. Verbindung gemäß Ansprüchen 1-19, worin eines aus R¹, R², R³ und R⁴ eine Bindung mit X bildet und die anderen aus R¹, R², R³, R⁴ und R⁵ und R⁹-R¹² aus Wasserstoff, Halogen, Cyano, Nitro, Amino, C₁₋₆-Alkyl, C₁₋₆-Alkoxy, C₁₋₆-Alkylthio, Hydroxy und Trifluormethyl ausgewählt sind und R Wasserstoff, C₁₋₆-Alkyl oder C₁₋₆-Alkylcarbonyl ist.

21. Verbindung gemäß Anspruch 20, worin R² oder R³ eine Bindung mit X bildet.

22. Verbindung gemäß Ansprüchen 1-19, worin X N ist.

23. Verbindung gemäß Ansprüchen 1-19, worin X C ist.

24. Verbindung gemäß Ansprüchen 1-19, worin X CH ist.

25. Verbindung gemäß Ansprüchen 1-19, worin R⁶, R⁷ und R⁸ Wasserstoff sind.

26. Verbindung gemäß Ansprüchen 1-19, worin R, R¹-R⁵ und R⁹-R¹² Wasserstoff oder Halogen sind.

27. Verbindung gemäß Ansprüchen 1-19, worin W eine Bindung ist und n+m 2 bis 4 ist.

28. Verbindung gemäß Anspruch 1, die ausgewählt ist aus:
5-{4-[2-(2-Oxo-3,4-dihydro-2H-chinolin-1-yl)ethyl]piperazin-1-yl}-1H-indol,
5-{4-[3-(2-Oxo-3,4-dihydro-2H-chinolin-1-yl)propan-1-yl]piperazin-1-yl}-1H-indol,
5-{4-[4-(2-Oxo-3,4-dihydro-2H-chinolin-1-yl)butan-1-yl]piperazin-1-yl}-1H-indol,
6-{4-[3-(2-Oxo-3,4-dihydro-2H-chinolin-1-yl)propan-1-yl]piperazin-1-yl}-1H-indol,
6-{4-[4-(2-Oxo-3,4-dihydro-2H-chinolin-1-yl}butan-1-yl]piperazin-1-yl}-1H-indol,
5-{4-[3-(2-Oxo-3,4-dihydro-2H-chinolin-1-yl)propan-1-yl]-3,6-dihydro-2H-pyridin-4-yl}-1H-indol,
5-{4-[3-(2-Oxo-3,4-dihydro-2H-chinolin-1-yl)propan-1-yl]piperidin-4-yl}-1H-indol,
5-{4-[4-(3-Oxo-3,4-dihydro-2H-1,4-benzoxazin-4-yl)butan-1-yl]piperazin-1-yl}-1H-indol,
5-{4-[3-(1-Oxo-3,4-dihydro-1H-chinolin-2-yl)propan-1-yl]piperazin-1-yl}-1H-indol,
5-{4-[4-(1-Oxo-3,4-dihydro-1H-chinolin-2-yl)butan-1-yl]piperazin-1-yl}-1H-indol,
5-{4-[3-(3,4-Dihydro-2H-chinolin-1-yl)propan-1-yl]piperazin-1-yl}-1H-indol,
5-{4-[3-(3,4-Dihydro-2H-chinolin-1-yl)propan-1-yl]piperazin-1-yl}-1H-indol,
5-{4-[3-(3,4-Dihydro-1H-isochinolin-2-yl)-3-oxopropan-1-yl]piperazin-1-yl}-1H-indol und
5-{4-[4-(3,4-Dihydro-1H-isochinolin-2-yl)-4-oxobutan-1-yl]piperazin-1-yl}-1H-indol, oder pharmazeutisch akzeptable Salze davon.

29. Pharmazeutische Zusammensetzung, die **dadurch gekennzeichnet ist, daß** sie eine Verbindung gemäß einem der Ansprüche 1 bis 28 in einer therapeutisch wirksamen Menge zusammen mit einem oder mehreren pharmazeutisch akzeptablen Trägern oder Verdünnungsmitteln umfaßt.

30. Verwendung einer Verbindung gemäß einem der Ansprüche 1 bis 28 zur Herstellung eines Medikaments, das nützlich ist in der Behandlung von positiven und negativen Symptomen von Schizophrenie, anderen Psychosen, Angststörungen wie allgemein Angststörung, Panikstörung und Zwangsstörung, Depression, Aggression, kognitiven Störungen, durch herkömmliche Antipsychotika induzierten Nebenwirken, Migräne, Aufmerksamkeitsdefizithyperaktivitätsstörung und in der Verbesserung von Schlaf.

31. Verbindung gemäß einem der Ansprüche 1 bis 28 zur Verwendung in einem Verfahren zur Behandlung von positiven und negativen Symptomen von Schizophrenie, anderen Psychosen, Angststörungen wie allgemeine Angststörung, Panikstörung und Zwangsstörung, Depression, Aggression, kognitiven Störungen, durch herkömmliche Antipsychotika induzierten Nebenwirkungen, Migräne, Aufmerksamkeitsdefizithyperaktivitätsstörung und in der Verbesserung von Schlaf, umfassend das Verabreichen einer therapeutisch akzeptablen Menge einer Verbindung gemäß einem der Ansprüche 1 bis 28.

## Revendications

1. Dérivé d'indole de formule 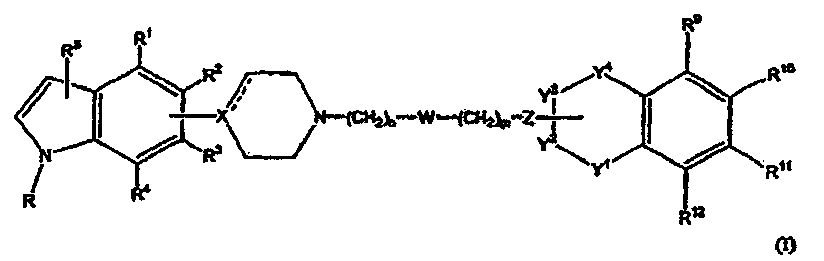 dans laquelle
a) Y¹ est N, qui est lié à Z, Z et Y² sont choisis parmi CH₂, CO, CS, SO et SO₂; à condition qu'au moins l'un des Z et Y² soit CH₂; Y³ est O, S ou CHR⁷ et Y⁴ est O, S ou CHR⁸, à condition qu'un seul des Y³ et Y⁴ soit O ou S;
b) Y² est N, qui est lié à Z, Z et Y¹ sont choisis parmi CH₂, CO, CS, SO et SO₂; à condition qu'au moins l'un des Z et Y¹ soit CH₂; Y³ est CHR⁷ et Y⁴ est O, S ou CHR⁸;
c) Y² est N, qui est lié à Z, Z et Y³ sont choisis parmi CH₂, CO, CS, SO et SO₂; à condition qu'au moins l'un des Z et Y³ soit CH₂; Y¹ est CHR⁶ et Y⁴ est O, S ou CHR⁸;
W est une liaison, O, S, CO, CS, SO ou SO₂;
X est C, CH ou N, à condition que, lorsque X est C, la ligne pointillée indique une liaison et que, lorsque X est N ou CH, la ligne pointillée indique une absence de liaison;
n est 0-5, m est 0-5 et n + m est égal à 1-6; à condition que n ≥ 2 et m ≥ 1 lorsque W est O ou S; et que n ≥ 1 et m ≥ 1 lorsque W est CO, CS, SO ou SO₂;
l'un des R¹, R², R³ et R⁴ forme une liaison vers X et les R¹, R², R³, R⁴ restants et R⁵ et R⁹-R¹² sont choisis indépendamment parmi l'hydrogène, les halogènes et les restes cyano, nitro, amino, hydroxy, (alkyl en C₁-C₆)amino, di(alkyl en C₁-C₆)amino, alkyle en C₁-C₆, alcényle en C₂-C₆, alcynyle en C₂-C₆, alcoxy en C₁-C₆, alkylthio en C₁-C₆, alkyle en C₁-C₆ substitué par hydroxy ou thiol, cycloalkyle en C₃-C₈, (cycloalkyl en C₃-C₈)-(alkyle en C₁-C₆), acyle, thioacyle, trifluorométhyle, trifluorométhylsulfonyle et (alkyl en C₁-C₆)sulfonyle;
R⁶, R⁷ et R⁸ sont l'hydrogène ou un halogène;
R est l'hydrogène ou un reste alkyle en C₁-C₆, alcényle en C₂-C₆, alcynyle en C₂-C₆, alkyle en C₁-C₆ substitué par hydroxy ou thiol, cycloalkyle en C₃-C₈, (cycloalkyl en C₃-C₈)-(alkyle en C₁-C₆), acyle, thioacyle, trifluorométhylsulfonyle ou (alkyl en C₁-C₆)sulfonyle;
ou ses sels pharmaceutiquement acceptables.

2. Composé selon la revendication 1, dans lequel Y¹ est N, qui est lié à Z, Z et Y² sont choisis parmi CH₂, CO, CS, SO et SO₂; à condition qu'au moins l'un des Z et Y² soit CH₂; Y³ est O, S ou CHR⁷ et Y⁴ est O, S ou CHR⁸, à condition qu'un seul des Y³ et Y⁴ soit O ou S.

3. Composé selon la revendication 2, dans lequel Y¹ est N, qui est lié à Z, Z et Y² sont choisis parmi CH₂ et CO; à condition qu'au moins l'un des Z et Y² soit CH₂; Y³ est CHR⁷ et Y⁴ est O, S ou CHR⁸.

4. Composé selon la revendication 2, dans lequel Y¹ est N, qui est lié à Z, Z est CH₂, Y² est CO, Y³ est CHR⁷ et Y⁴ est O, S ou CHR⁸.

5. Composé selon la revendication 2, dans lequel Y¹ est N, qui est lié à Z, Z est CH₂, Y² est CO, Y³ est CH₂ et Y⁴ est CH₂.

6. Composé selon la revendication 2, dans lequel Y¹ est N, qui est lié à Z, Z et Y² sont CH₂; Y³ est CHR⁷ et Y⁴ est O, S ou CHR⁸.

7. Composé selon la revendication 2, dans lequel Y¹ est N, qui est lié à Z, Z est CO, Y² est CH₂, Y³ est CHR⁷ et Y⁴ est O, S ou CHR⁸.

8. Composé selon la revendication 1, dans lequel Y² est N, qui est lié à Z, Z et Y¹ sont choisis parmi CH₂, CO, CS, SO et SO₂; à condition qu'au moins l'un des Z et Y¹ soit CH₂; Y³ est CHR⁷ et Y⁴ est O, S ou CHR⁸.

9. Composé selon la revendication 8, dans lequel Y² est N, qui est lié à Z, Z et Y¹ sont choisis parmi CH₂ et CO; à condition qu'au moins l'un des Z et Y¹ soit CH₂; Y³ est CHR⁷ et Y⁴ est O, S ou CHR⁸.

10. Composé selon la revendication 8, dans lequel Y² est N, qui est lié à Z, Z est CH₂ et Y¹ est CO; Y³ est CHR⁷ et Y⁴ est O, S ou CHR⁸.

11. Composé selon la revendication 8, dans lequel Y² est N, qui est lié à Z, Z est CH₂ et Y¹ est CO; Y³ est CH₂ et Y⁴ est CH₂.

12. Composé selon la revendication 8, dans lequel Y² est N, qui est lié à Z, Z et Y¹ sont CH₂; Y³ est CHR⁷ et Y⁴ est O, S ou CHR⁸.

13. Composé selon la revendication 8, dans lequel Y² est N, qui est lié à Z, Z est CO et Y¹ est CH₂; Y³ est CHR⁷ et Y⁴ est O, S ou CHR⁸.

14. Composé selon la revendication 8, dans lequel Y² est N, qui est lié à Z, Z est CO et Y¹ est CH₂; Y³ est CH₂ et Y⁴ est CH₂.

15. Composé selon la revendication 1, dans lequel Y² est N, qui est lié à Z, Z et Y³ sont choisis parmi CH₂, CO, CS, SO et SO₂ à condition qu'au moins l'un des Z et Y³ soit CH₂; Y¹ est CHR⁶ et Y⁴ est O, S ou CHR⁸.

16. Composé selon la revendication 15, dans lequel Y² est N, qui est lié à Z, Z et Y³ sont choisis parmi CH₂ et CO; Y¹ est CHR⁶ et Y⁴ est O, S ou CHR⁸.

17. Composé selon la revendication 15, dans lequel Y² est N, qui est lié à Z, Z est CH₂ et Y³ est CO; Y¹ est CHR⁶ et Y⁴ est O, S ou CHR⁸.

18. Composé selon la revendication 15, dans lequel Y² est N, qui est lié à Z, Z et Y³ sont CH₂; Y¹ est CHR⁶ et Y⁴ est O, S ou CHR⁸.

19. Composé selon la revendication 15, dans lequel Y² est N, qui est lié à Z, Z est CO et Y³ est CH₂; Y¹ est CHR⁶ et Y⁴ est O, S ou CHR⁸.

20. Composé selon les revendications 1-19, dans lequel l'un des R¹, R², R³ et R⁴ forme une liaison vers X et les R¹, R², R³, R⁴ restants et R⁵ et R⁹-R¹² sont choisis parmi l'hydrogène, les halogènes et les restes cyano, nitro, amino, alkyle en C₁-C₆, alcoxy en C₁-C₆, alkylthio en C₁-C₆, hydroxy et trifluorométhyle, et R est un hydrogène ou un reste alkyle en C₁-C₆ ou (alkyl en C₁-C₆)carbonyle.

21. Composé selon la revendication 20, dans lequel R² ou R³ forme une liaison vers X.

22. Composé selon les revendications 1-19, dans lequel X est N.

23. Composé selon les revendications 1-19, dans lequel X est C.

24. Composé selon les revendications 1-19, dans lequel X est CH.

25. Composé selon les revendications 1-19, dans lequel R⁶, R⁷ et R⁸ représentent l'hydrogène.

26. Composé selon les revendications 1-19, dans lequel R, R¹-R⁵ et R⁹-R¹² représentent l'hydrogène ou un halogène.

27. Composé selon les revendications 1-19, dans lequel W est une liaison et n + m est égal à 2 à 4.

28. Composé selon la revendication 1, qui est choisi parmi
le 5-{4-[2-(2-oxo-3,4-dihydro-2H-quinoléin-1-yl)éthyl]pipérazin-1-yl}-1H-indole,
le 5-{4-[3-(2-oxo-3,4-dihydro-2H-quinoléin-1-yl)propan-1-yl]pipérazin-1-yl}-1H-indole,
le 5-{4-[4-(2-oxo-3,4-dihydro-2H-quinoléin-1-yl)butan-1-yl]pipérazin-1-yl}-1H-indole,
le 6-{4-[3-(2-oxo-3,4-dihydro-2H-quinoléin-1-yl)propan-1-yl]pipérazin-1-yl}-1H-indole,
le 6-{4-[4-(2-oxo-3,4-dihydro-2H-quinoléin-1-yl)butan-1-yl]pipérazin-1-yl)-1H-indole,
le 5-{4-[3-(2-oxo-3,4-dihydro-2H-quinoléin-1-yl)propan-1-yl]-3,6-dihydro-2H-pyridin-4-yl}-1H-indole,
le 5-{4-[3-(2-oxo-3,4-dihydro-2H-quinoléin-1-yl)propan-1-yl]pipéridin-4-yl}-1H-indole,
le 5-{4-[4-(3-oxo-3,4-dihydro-2H-1,4-benzoxazin-4-yl)butan-1-yl]pipérazin-1-yl}-1H-indole,
le 5-{4-[3-(1-oxo-3,4-dihydro-1H-quinoléin-2-yl)propan-1-yl]pipérazin-1-yl}-1H-indole,
le 5-{4-[4-(1-oxo-3,4-dihydro-1H-quinoléin-2-yl)butan-1-yl]pipérazin-1-yl}-1H-indole,
le 5-{4-[3-(3,4-dihydro-2H-quinoléin-1-yl)propan-1-yl]pipérazin-1-yl}-1H-indole,
le 5-{4-[3-(3,4-dihydro-2H-quinoléin-1-yl)propan-1-yl]pipérazin-1-yl}-1H-indole,
le 5-{4-[3-(3,4-dihydro-1H-isoquinoléin-2-yl)-3-oxopropan-1-yl]pipérazin-1-yl}-1H-indole, et
le 5-{4-[4-(3,4-dihydro-1H-isoquinoléin-2-yl)-4-oxobutan-1-yl]pipérazin-1-yl}-1H-indole,
ou leurs sels pharmaceutiquement acceptables.

29. Composition pharmaceutique **caractérisée en ce qu'**elle comprend un composé selon l'une quelconque des revendications 1 à 28 en une quantité thérapeutiquement efficace avec un ou plusieurs supports ou diluants pharmaceutiquement acceptables.

30. Utilisation d'un composé selon l'une quelconque des revendications 1 à 28 pour la préparation d'un médicament utile dans le traitement des symptômes positifs et négatifs de la schizophrénie, d'autres psychoses, de l'anxiété, comme l'anxiété généralisée, la panique et les troubles obsessionnels compulsifs, de la dépression, de l'agressivité, des troubles cognitifs, des effets secondaires induits par des agents antipsychotiques classiques, de la migraine, de l'hyperactivité avec déficit de l'attention et pour améliorer le sommeil.

31. Composé selon l'une quelconque des revendications 1 à 28 à utiliser dans un procédé de traitement des symptômes positifs et négatifs de la schizophrénie, d'autres psychoses, de l'anxiété, comme l'anxiété généralisée, la panique et les troubles obsessionnels compulsifs, de la dépression, de l'agressivité, des troubles cognitifs, des effets secondaires induits par des agents antipsychotiques classiques, de la migraine, de l'hyperactivité avec déficit de l'attention et pour améliorer le sommeil, comprenant l'administration d'une quantité thérapeutiquement acceptable d'un composé selon l'une quelconque des revendications 1 à 28.
